# EUROPEAN PATENT SPECIFICATION

(11) **EP 1 062 517 B1**
(45) Date of publication and mention of the grant of the patent: **01.03.2006**
(21) Application number: 98941283.8
(22) Date of filing: 16.06.1998
(51) Int. Cl.: G01N 33/68

(54) **DIAGNOSIS OF SPONGIFORM ENCEPHALOPATHIES USING PRIONINS**
DIAGNOSE SPONGIFORMER ENCEPHALOPATHIEN MIT PRIONINEN
USAGE DES PRIONINES POUR LE DIAGNOSTIC DES ENCEPHALOPATHIES SPONGIFORMES

(30) Priority: 16.06.1997 CA 2206774
(43) Date of publication of application: 27.12.2000
(73) Proprietor: BERGMANN, Johanna, E., D-22587 Hamburg (DE); Preddie, Rick, E., 22587 Hamburg (DE)
(72) Inventor: BERGMANN, Johanna, E., D-22587 Hamburg (DE); Preddie, Rick, E., 22587 Hamburg (DE)
(86) International application number: PCT/EP1998/003609
(87) International publication number: WO 1999/004237

(56) References cited:
- WO-A-94/24554
- WO-A-98/07850
- BIOLOGICAL ABSTRACTS, Philadelphia PA USA; abstract no. PREV199699174983, see title XP002098215 & J.E. BERGMANN ET AL.: "ALZAS, a protein found in brain and blood of humans with Alzheimer's disease (AD) but not in normal humans, appears to be the causative biochemical factor of all forms of AD: " NEUROBIOLOGY OF AGING, vol. 17, no. 4 supplement, 24 July 1996, pages S14-S15, Washington DC USA

## Description

### FIELD OF THE INVENTION

This invention is related to diagnostic and therapeutic molecules for the detection, prevention of bovine spongiform encephalopathy (BSE), scrapie disease (scrapie) and Creutzfeldt-Jakob syndrome (CJS). Specifically, the invention relates to three closely related proteins BSAS, SCRAPAS and CJAS which are implicated in causing BSE, scrapie and CJS, to antagonists of these proteins, to diagnostic reagents to detect these proteins in clinical samples and food. It is also related to the use of homologues of these proteins from hamsters and mice which are useful for developing and testing methods for use with vaccines and other agents for therapeutic value in treatment of humans and animals for TSE diseases.

### BACKGROUND OF THE INVENTION

Prion proteins (PrPs) are a family of very closely related proteins which are found in a number of allelic forms in the membrane of certain populations of brain neurons of all animals. PrPs are expressed in lymphoreticular system and replicated in spleen and other lymphoreticular tissues (Kimberin R.H. and Walker C.A. Virus Res. 12:201-211 (1989). The alteration of the molecular configuration (folding) of a PrP by an unknown mechanism which converts these proteins into infectious particles "prions" (PrPsc) is associated with a group of diseases called "Transmissible spongiform encephalopathies" (TSE) (Prusiner S. B. Science 252:1515-1522 (1991); Prusiner S.B. Ed., Current topics in Microbiology and Immunology 207 (1996)); (Westaway D et al., Trends. Microbiol. 3:141-147 (1995)); (Goldfarb L.G. and Brown P. Annu. Rev: Med. 46:57-65 (1965)).

Three members of the TSE family are of great economic and medical concern; these include Bovine spongiform encephalopathy (BSE) in cows, scrapie in sheep and Creutzfeldt-Jakob syndrome (CJS) in humans. Pathology of TSE involve nerve cell dysfunction which leads to fatal neurodegeneration; TSEs are characterized by symmetrical vacuolation of neurons and neuropil and accumulation of PrPSc around neurons. The latter phenomenon is believed to be the cause of the phenotype of the disease in the affected animal or human (Darcel C. Vet. Res Commun. 19:231-252 (1995)).

Prions are believed to be self replicating proteins which in their altered configuration are resistant to destruction by proteolytic enzymes and heating conditions which usually destroy most proteins. The infectious prion is believed to be transmissible across species. Nevertheless, it has not been satisfactorily explained how a brain resident protein which, so far has not been demonstrated in biological fluids, can be transmitted from animal to animal within a grazing herd of cattle. Also, it is a puzzle that an endogenous protein auto-converts from a harmless, useful, form to a highly pathogenic infective form; many exotic hypotheses have been proposed to explain these phenomena.

However a few plausible hypotheses have provided insight into some aspects of the above mentioned puzzle, e.g., (i) it has been shown that PrPsc can be detected in the tonsils of animals with BSE and scrapie and presumably, animals presymptomatic for the above diseases (Schreuder B.E et al., Nature 381:563 (1996); (ii) it has been suggested that prions could be receptors that ushers an unknown virus or other infectious agents into cells (Brown P. (quoted in special news report) "Putting Prions to the Test" Science 273:184-189 1996)); (iii) experiments in mice appear to indicate that PrP binds to a chaperon protein (protein X) which catalyses the formation of PrPSc (Telling G.C. Cell 83:79-90 (1995) ) ; (iv) that an unidentified factor is responsible for BSE and prions act as host-adapting agent for the factor (Lasmezas C.I. et al Science 275:402-405 (1997), and (iv) that a frame shift translation in scrapie PrP mRNA may play a role in conversion of PrP to PrPsc, (Wills P. R. et al. Microbiol. pathogenesis 6:235-249 (1989); Kiyotoshi K. et al.; Proc. natl. Acad. Sci. USA 94:10069-10074 (1998).

There is an intriguing relationship between Scrapie in sheep, BSE in cows and CJS in humans; it is believed that BSE is initiated by the scrapie prion and some forms of CJD are initiated by eating BSE infected cows, particularly brain. The latter appeared to be experimentally been supported by the discovery, recently, of what has been called a BSE signature molecule in humans who contract a "new variant of CJD (Collinge, J et al., Nature 283:685-690 1996. Nevertheless despite a large international effort to understand the pathogenesis of TSE, and to develop non invasive widely applicable methods for presymptomatic detection of these diseases, which in the case of BSE and scrapie have calamitous effects on commercial activity and on the health of the population at large, substantial progress has not been made in these directions.

### SUMMARY OF THE INVENTION:

The object of the present invention was to find other proteins which were expressed in a disease-specific manner, and which might interact with and convert PrP to PrPsc. We reasoned that such proteins which would occur in body fluids, would be highly specific ante-mortem diagnostic markers in pre symptomatic cows, sheep and humans and they would be useful as therapeutic targets to manage the symptoms of TSE diseases in animals and humans. Furthermore, we aimed to invent a method/methods which would detect cross infection of humans and other animals by such agents that cause BSE and scrapie.

According to the invention the solution to the problem was to use the implied relationship between human Alzheimer's disease (AD) and the prion diseases, especially the relationship of a protein ALZAS, which we have discovered to be the potential causative factor of all forms of AD. ALZAS (Bergmann J. E. et al., Neurobiology of Aging 17: S14 (1996), a protein encoded and expressed within the human APP gene; the latter is strongly linked to Alzheimer's disease (AD). Post translational modification of the APP gene product the "ß amyloid precursor protein" the ß amyloid protein (Aß). Aß plays a central role in the pathophysiology of AD (Scheuner D. et al., Nature Medicine 2:864-870 (1996)). Recently it was shown that protein products of frame shift mutations in the APP gene and the ubiquitin ß gene might be involved in the pathology of Alzheimer's disease and Down syndrome (van Leeuwen F. et al., Science 279 :242-247, (1998). ALZAS which is made up of Aß, the APP protein transmembrane signal sequence and a unique intron encoded c-terminal sequence, is detected in brain, blood and saliva of all humans with Alzheimer's disease (AD) and has the predicted biochemical characteristics to initiate the clinical symptoms of AD (Bergmann J.E. et al., Neurobiology of Aging 17:S14 (1996)). The pathophysiological similarities between AD and the TSEs, particularly CJD, and the implied relationship of ALZAS to APP/Aß led us to search for a molecule similar to ALZAS within the prion protein genes of cattle, sheep and humans, and in mice and hamsters. The two rodent species which have been used extensively by scientist wanting to model TSE diseases. Using the method which we call disease "gene discovery by positional searching" (DGDPS), (see Bergmann J. and Preddie E. "WIPO" publications No# WO98/07850 and No#WO98/07851). Our search led to the discovery of proteins BSAS, SCRAPAS and CJAS which are encoded and expressed from within the PrP genes of cattle, sheep and humans respectively: Additionally we discovered MPAS and HAMPAS, which were encoded within the PrP genes of mice and hamsters. Since these proteins were discovered within the chromosomal region encoding prion protein genes we named them "prionins". Following is a brief description of DGDPS as it was applied to the discovery of prionins

### Relationship of BSAS and SCRAPAS to BSE and Scrapie

These proteins appear to be specifically related to the development of BSE in cattle and scrapie in sheep. BSAS and SCRAPAS appear in the blood of all animals with clinical symptoms of the disease and in a significant percentage of animals that have been exposed to the disease, but not usually in animals that have not been exposed to the disease. In addition, animals exposed to the disease produce specific antibody (endogenous antibody) directed against BSAS or SCRAPAS, which can be detected presymptomatic in the sera of affected animals. The concentration of the endogenous antibody in the serum of animals with clinical symptoms of the disease appears to be generally lower than that of the animals without clinical symptoms of the disease, which led us to speculate that there may be a weakening of a subjects immune defense at initiation of the disease.

### BSAS and SCRAPAS

BSAS and SCRAPAS are relatively small ß-sheet derived proteins expressed from within the prion protein genes of cows and sheep. These proteins are extremely hydrophobic, have the potential to bind strongly to the prion proteins and to convert them into the β-conformation. They also have sequence homologies to one region of the prion protein which has been shown by others to be involved in the interaction of the so called 'protein X' to the sheep prion protein. In addition to having the possibility of penetrating membranes, BSAS and SCRAPAS have structural similarities to powerful DNA-binding proteins and might autoactivace their own expression. They have about 60 % overall similarity with each other. More than 20 % of the amino acids are tryptophan as compared to the average tryptophan content < 1% for all proteins for which the amino acid sequence is known.

Two epitopes (BSAS epi and SCRAPAS epi, 14 amino acids each) were selected, chemically synthesized and used to produce polyclonal antibodies against BSAS (BSAS pcAb) and SCRAPAS (SCRAPAS pcAb) in rabbits. Two sub-epitopes from within BSAS epi (BSAS mepi) and SCRAPAS epi (SCRAPAS mepi), were synthesized, amidated at the c-terminal end and used to affinity purify a specific population of antibodies from BSAS pcAb and SCRAPAS pcAb, respectively. These antibodies, and the mepi epitopes, were used in the ELISA tests which are described below.

### ELISA Tests

Two types of ELISAs have been developed. One detects in serum the antigens BSAS respectively SCRAPAS, the other detects endogenous antibodies (specific IgG) against BSAS and SCRAPAS mepi epitopes. The specificity of all epitopes was confirmed both in commercial scale isolation of specific populations of IgG's from BSAS pcAb and SCRAPAS pcAb, and in the isolation of endogenous IgG from serum samples of selected BSE-infected cattle and scrapie-infected sheep. Potential cross-reacting antigenic epitopes were not found in the protein data bases presently available.

### Treatment of serum samples for testing.

BSAS and SCRAPAS are extremely sensitive to freezing. Freezing overnight at -20°C reduces the reaction of a positive sample by 70-80%; freezing at -80 °C eliminates the reaction completely. We believe that this high sensitivity to freezing is due to the high tryptophan content of the proteins, and the secondary structure enforced on the proteins by the tandem arrangements of several tryptophan triads in the proteins. The effect of freezing, although not as great for the Ab trap ELISA as for the Ag trap ELISA, seriously affects both ELISAs (antigen molecules with altered confirmation bind irreversible to endogenous IgG in positive sera which sometimes cause erratic ELISA results in the Ab trap assay). Therefore, frozen samples are not suitable for use in the ELISAs. Serum samples must be prepared from freshly drawn blood and stored at 4°C for such investigations.

### Outline of the Tests

### The Antigen Trap Test

The wells of micro titre plates were coated with 100 µl of carbonate buffer, pH 9.6, containing 5 µg/ml mepi epitope-specific antibody, at 4°C over night. The plates were washed 3 x with ELISA washing buffer containing 0.05 % Tween-20. Serum samples, 5 µl, were added to 45 µl of ELISA washing buffer containing 0.075 % Tween-20, and incubated at 37°C for 30 minutes. Wells were washed 5 x with the 0.075 % Tween-20 washing buffer. 50 µl of second antibody (affinity purified SCRAPAS/BSAS-specific antibody, labelled with horseradish peroxidase (HRP)) diluted 1/40 in washing buffer containing 0.05 % Tween-20, was added to each well and the plates were incubated at 37°C for 45 minutes. The plates were again washed 5 x with washing buffer containing 0.075 % Tween-20. 50 µl substrate solution (Sigma OPD tablet set) was added to each well; colour development was allowed for 30 minutes in the dark, and the plates were read at 492 nm.

### The Antibody Trap Test

The wells of micro titre plates were coated with 100 µl of carbonate buffer, pH 9.6, containing 10 µg/ml of each, BSAS mepi and SCRAPAS mepi epitopes, at 4°C over night. The plates were washed 3 x with ELISA washing buffer containing 0.05 % Tween-20. Serum samples, 1.5 µl, were added to 48.5 µl of ELISA washing buffer containing 0.075 % Tween-20, and incubated at 37°C for 30 minutes. Wells were washed 5 x with the 0.075 % Tween-20 washing buffer. 50 µl of second antibody (mixture of anti-bovine IgG and anti-sheep IgG antibodies (γ-chain specific, labelled with horseradish peroxidase (HRR)) diluted 1/12000 in washing buffer containing 0.05 % Tween-20, was added to each well and the plates were incubated at 37°C for 45 minutes. The plates were again washed 5 x with washing buffer containing 0.075 % Tween-20. 50 µl substrate solution (Sigma OPD tablet set) was added to each well; colour development was allowed for 30 minutes in the dark, and the plates were read at 492 nm.

### Description of DGDPS procedure.

In general, first we identified a gene closely related to a gene already genetically or otherwise linked to a certain disease, then isolated the mRNA transcribed from the gene from disease tissue or patient's blood, then synthesized cDNA from the isolated mRNA with reverse transcriptase, then amplified the novel cDNA with specific primers which flanked the entire coding region of the cDNA, then we identified the cDNA from the size following electrophoresis on agarose gel, and finally isolated the unique cDNA from the agarose gel. This allowed us to select out the desired molecule, if it was expressed, without having to probe several million cDNA clones.

The procedure as used in the present invention and the results obtained are described in the following examples.

### Example 1 (discovery of cattle PrP prionin BSAS)

(1) We examined the sequenced regions within the bovine prion protein gene locus and selected potential complete orf's, i.e. with acceptable translation initiation sequences (see Kozak, M. Nucleic Acid Res. 12:857-872 (1984)) and translation termination stop codons (TAA, TAG or TGA) in place,
(3) then, orf's fulfilling the above two characteristics were translated into putative protein sequences using the universal code,
(4) then we analyzed the putative protein with our proprietary computer assisted protein finger printing technology and obtained information about the potential biochemical characteristics of the deduced proteins,
(5) next the biochemical characteristics of the deduced proteins were correlated with the known biochemical characteristics of BSE.

Then we determined if the protein was expressed: in order to do this two potential epitopes were identified/selected within the amino acid sequence at n-terminal and c-terminal of the deduced protein using the method of Hopp and Woods, K.R. Proc. Natl. Acad. Sci. USA 78:3824-3828 (1981). The sequences were compared to sequences in databases and those which appeared to have no homologue within the databases were selected. Mono-specific polyclonal rabbit antibodies were prepared against these and purified by immunoaffinity chromatography on CNBr-activated sepharose 4B (Pharmacia) according to a procedure which combined the first section of the recommendation of the manufacturer and the second section as described in Current Protocols in Molecular Biology (Vol 1) Ausubel, F.M. et al (ed) John Wiley & Sons NY. NY. 1991 and polyclonal IgG was coupled to horse radish peroxidase. An enzyme-based immunoassay format "sandwich ELISA" (described in "ELISA and other Solid Phase Immunoassays" (Kemeny, D.M. et al. (eds) John Wiley & sons, N.Y, N.Y (1988), was used to probe serum, blood, saliva from BSE positive cows for BSAS using anti-BSAS IgG -HRP in a colorimetric reaction with "Sigma OPD" as substrate. The procedure is outlined in section above.

### Example 2

### Detection of BSAS, SCRAPAS and CJAS in clinical and other samples.

Prionin proteins were detected using the ELISAs described above. Since some populations ft the polyclonal anti-TSE antibodies cross react with domains of the major epitopes of each TSE species, with varying degrees of sensitivity, anti-SCRAPAS-HRP is used as the second antibody for detecting both BSAS and SCRAPAS, whereas either anti-SCRAPAS HRP or anti-CJAS-HRP is used as the second antibody for detecting CJAS.

### Example 3

Using another approach, which is not suitable for use as a routine test method (with BSAS as the example): BSAS protein was isolated from ~100 ul of serum, from an infected cow on a anti-BSAS antibody immuno-affinity column. The isolated protein was subjected to SDS gel electrophoresis as described by Schägger, H. and von Jabow, G.I. Anal. Biochem. 166:368-379 (1987) or by non-SDS PAGE. Following electrophoresis the proteins were subjected to Western blotting or spotted onto nylon membrane and treated with the affinity purified antibody. Interaction of the antibody with the protein bound to the membrane was visualized with a chemiluminescent kit purchased from Bio-Rad Inc. according to the manufacturer's instructions (also see Blake M.S. et al. Anal biochem. 136:175-179 (1984)). MOPAS and HAMPAS were discovered using the same procedure; however, the expression of these two proteins in animals with experimental TSE disease have not been investigated so far.

### Example 4

We have demonstrated, in the case of BSAS and SCRAPAS, a specific association (100%) of the proteins with animals confirmed with the disease or exposed in any manner to the disease; whereas the proteins were not associated with animals which have never been exposed to the disease. BSAS and SCRAPAS was detected in serum of all animals clinically positive for the diseases and in the majority of animal that had no demonstrated clinical symptoms of the disease which came from herds that had even a single case of the disease. CJAS was detected in serum of two CJD victims in one of which was positive for both CJD and Alzheimer's disease.

### DESCRIPTION OF THE PREFERRED EMBODIMENTS:

The success of the present invention derived from our ability to find alternative genes encoding potentially pathogenic proteins BSAS, SCRAPAS and CJAS within the PrP genes of cattle, sheep and humans. The discovery of equivalent genes in mice and hamsters contributed to formulating our model for TSE.

### DETAILS OF THE INVENTION:

In detail the invention provides the following: (i) five protein molecules substantially free of natural contaminants, that encode a protein selected from BSAS, SCRAPAS, CJAS and MPAS and HAMPAS In particular the invention provides the aforementioned protein molecules wherein the sequence is SEQ ID:NO 1, SEQ ID:NO 2 SEQ ID:NO 3, SEQ ID:NO 4 and SEQ ID:NO 5.
(ii) A method for detecting latent CJD, BSE and scrapie in humans, cows and sheep respectively by using the ELISA method described above to detect endogenous IgG directed against an epitope in each protein BSAS (SEQ ID:NO 1); SCRAPAS (SEQ ID:NO 2) and CJAS (SEQ ID:NO 3) in blood of animals and humans respectively, that show no symptoms of the disease.
(iii) a method for detecting cross infection of animals by animals and human by animals by method described in (ii) above to detect the specific anti-prionin antibody in the blood of a subject.
(iii) A method of detecting contamination of meat, meat products and blood products by, BSAS (SEQ ID:NO 1), SCRAPAS (SEQ ID:NO 2) and CJAS (SEQ ID:NO 3), using one or both the ELISA methods described above to detect either the prionin or traces of a species specific anti prionin IgG in the product tested.

### THE MOLECULES OF THE INVENTION

BSAS, SCRAPAS and CJAS ("prionins") are encoded and expressed from within bovine, sheep and human prion genes:

BSAS "SEQ ID:NO 1", is a 9.6 Kd basic protein containing 19.75% tryptophan (Trp) residues, a stretch of 22 amino acids (aa) which is a predicted positive DNA regulatory unit and which classifies the protein as a positive DNA regulator protein. The secondary structure of BSAS is organized as five (possibly seven) tandem, adjoining, β sheets each separated by a β turn. BSAS contains two regions of significant homology to regions of bovine prion protein between amino acids 90-150. The latter region has been suggested by others to be involved in the etiology of BSE in cows. It also has intriguing homology to growth hormone releasing hormone receptor.

SCRAPAS "SEQ ID:NO 2", is a 7.63 Kd basic protein. It contains 21.87 % Trp residues and the identical predicted positive DNA regulatory domain which is contained in BSAS. The secondary structure of SCRAPAS is organised as four or five equally spaced β sheets in a pattern almost identical to BSAS. It has no significant region of homology to the major sheep prion protein, but has moderate homology to the region (aa 90 - 150) described above in the bovine prion protein.

CJAS "SEQ ID:NO 3", is a 7.7 Kd basic protein. It contains 21.53 % Trp residues. CJAS has a Pro-rich N-terminal region and a sequence of 27 amino acid which contains a potential transmembrane helix, which classifies it as a transmembrane protein. The secondary structure of CJAS, which is similar to that of BSAS and SCRAPAS, is organised as four adjoining β pleats each separated by a β turn. The protein has a domain with significant homology to a domain in the malaria parasite merozoite membrane protein which is believed to promote vacuole formation which gives a spongiform appearance to infected erythrocytes (see Dluzewski A.R. et al J. cell Sci. 92:691-699 (1989)

MPAS "SEQ ID:NO 4 is a 5.2 Kd neutral protein. It contains a single tryptophan residue (< 0.5%). The secondary structure is organized as three equally separated β sheets separated by two broad alpha helical regions. It is not a β sheet structured protein.

HAMPAS "SEQ ID:NO 5", 1s a 4.79 Kd basic protein: It contains 26% tryptophan residues. It is basically a CJAS protein truncated at the N terminal. It shares an antigenic epitope with CJAS. The secondary structure is organised as three adjoining β pleats which are arranged in a similar way to β-sheet 3,4 and 5 of CJAS.

### BRIEF DESCRIPTION OF THE FIGURES:

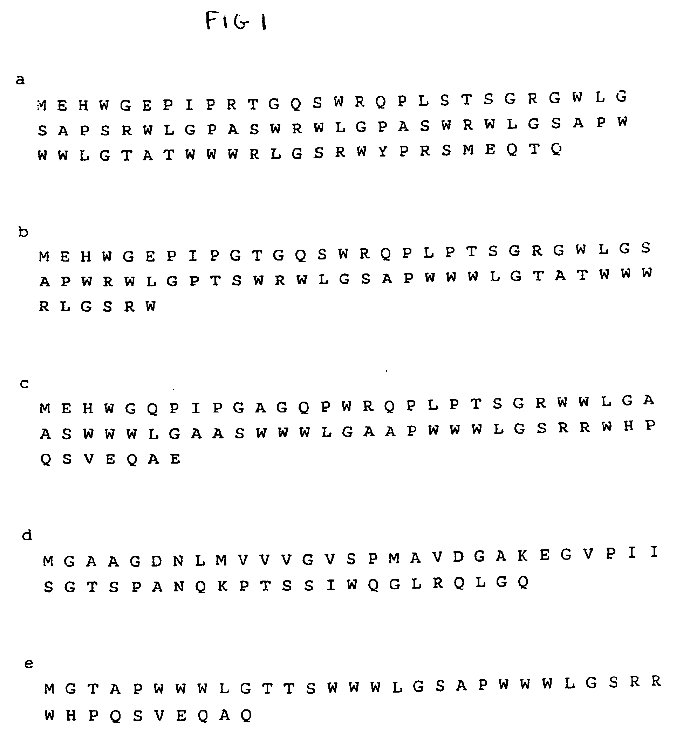

FIG. 1a (SEQ ID:NO 1) is the amino acid sequence of BSAS.
FIG. 1b (SEQ ID:NO 2) is the amino acid sequence of SCRAPAS.
FIG. 1c (SEQ ID:NO 3) is the amino acid sequence of CJAS.
FIG. 1d (SEQ ID:NO 4) is the amino sequence of MPAS
FIG. 1e (SEQ ID;NO 5) is the amino acid sequence of HAMPAS

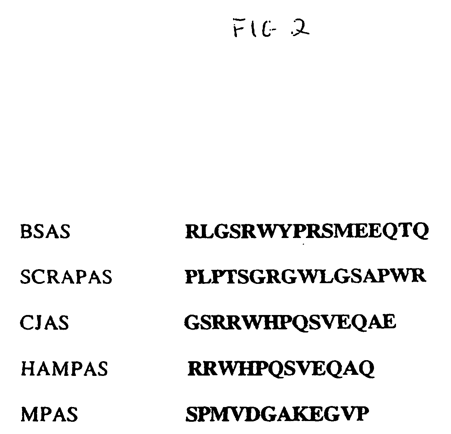

FIG. 2 a-c shows the sequence of antigenic epitopes in BSAS; SCRAPAS and CJAS respectively. SCRAPAS epitope appears in BSAS (13 of 14 amino acid residues in BSAS and 9 of 14 residues in CJAS; part of BSAS epitope appears in SCRAPAS and CJAS.
FIG. 3a ELISA detection of endogenous anti-BSAS IgG in serum of BSE positive and negative cattle.
FIG. 3b ELISA detection of endogenous anti-BSAS IgG in serum of BSE positive BSE negative and clinically negative cows from herds which had cases of BSE.
FIG. 3c ELISA detection of endogenous anti-SCRAPAS IgG in serum from scrapie positive and scrapie negative sheep..
FIG. 3d ELISA detection of endogenous anti-SCRAPAS IgG in serum of scrapie positive scrapie negative sheep and of clinically negative sheep that were exposed to scrapie.
FIG. 3e ELISA detection of CJAS protein in serum of patients with CJD and patients with other neurological diseases.
FIG. 3f ELISA detection of anti-CJAS shows endogenous IgG in serum of CJD patients and patients with a variety of other neurological diseases.
FIG. 3g immunoblot of a cationic, non SDS polyacrylamide gel which demonstrates the presence of BSAS and BSAS complexes to anti-BSAS IgG serum from a cow with BSE.
FIG. 4 sequences of the DNA binding domains of BSAS and SCRAPAS, (the sequences are 100% homologous) and the sequence of the membrane spanning alpha helix of CJAS.
FIG. 5a. evolutionary structural relationship between BSAS, SCRAPAS, CJAS, HAMPAS and MPAS suggested from cluster analysis of prions and prionins.
FIG. 5b the β-sheet structured characteristics of prionins.

### PHYSIOLOGICAL ROLE OF BSAS, SCRAPAS and CJAS IN THE NEUROPATHOLOGY OF BSE, SCRAPIE AND CJS.

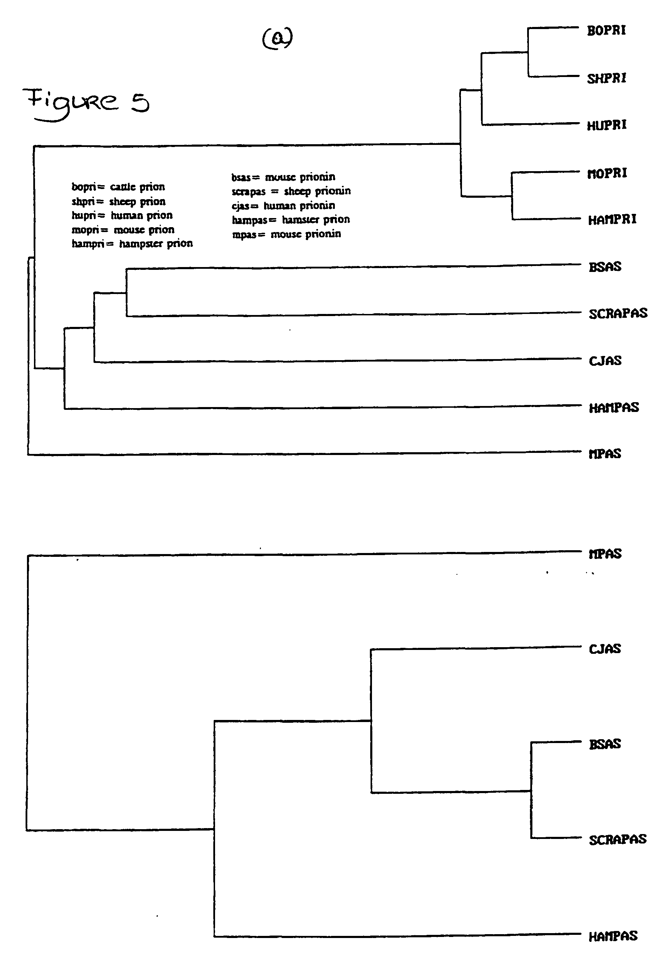

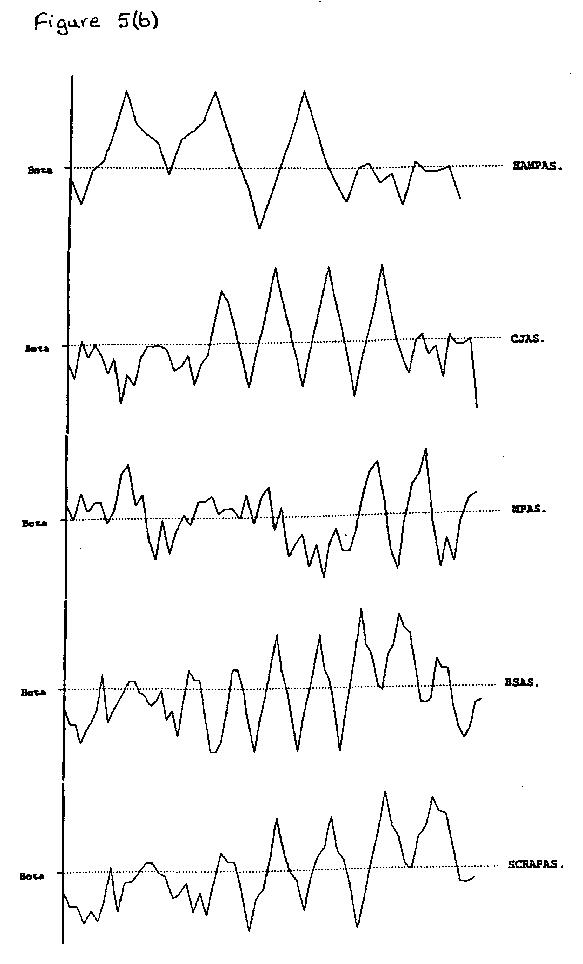

BSAS, SCRAPAS and CJAS ("prionins") are expressed from within the respective prion genes (as has been shown for the expression of frame shift proteins from within the human APP gene in Alzheimer's disease), structurally, they are closely related; however, the relationship does not appear to be as close as the relationship between the PrPs (see figure) which are not closely related to the prionins (figure 5a). Prionins are entirely β-sheet derived structures (figure 5b). In this respect they resemble snake and scorpion toxins and some insect definsins (pore forming proteins) which are predominantly β-sheet structures (Bontems, F. et al., Science 254:1521-1523 (1991)), but differ from these proteins because they lack cysteine residues; however prionins contain - 20 Trp residues which suggest that they are soluble in cell membranes and other cellular lipid containing structures. Furthermore, in prionins β-sheets are separated at regular intervals by β turns, and, except for very short sequences at c-terminal and n-terminal ends, the proteins are totally hydrophobic molecules; these structural characteristics are similar to functional domains of another family of proteins which interacts with and alters the secondary structure of other proteins (see PZD and PTB domains Zhou H, et al Nature Struct. Biol. 3:388-393 (1996)).

Structural characteristics mentioned above which indicate that prionins resemble families of pathogenic membrane seeking proteins suggest that prionins are pathogenic proteins. This is supported by the disease specific expression of BSAS, SCRAPAS and CJAS.

Pore forming proteins destroy cells by boring holes in the membranes,(Peitsch. M. et al., Mol. Immunol. 27 589-602 (1990)) In the cell cytoplasm they usually aggregate to become soluble. Although neither BSAS nor SCRAPAS contain a "computer defined" transmembrane signal; because they are, highly hydrophobic molecules, they might mimic pore forming proteins by entering plasma membranes using a hydrophobic wedge (see Hill, H.P. et al., Science 251:1481-1485 (1991)) made up mainly of Trp residues). Furthermore extremely high concentration of tryptophan residues in prionins indicate that they may be lipid soluble.

Current dogma holds that PrPs become infectious PrPscs when the secondary structure of the PrP "flips" from predominantly alpha helix to β pleats, and this molecular transformation causes the prions to leave the neuronal membrane and accumulate in extra neuronal spaces (Darcel, C. Vet. Res. Commun. 19:231-252 (1995)). As indicated above, the prionin proteins, can potentially bind to other proteins and enforce the β-plate configuration on the proteins they bind to. Like pore boring proteins prionins can also use the interacting protein as a chaperon for transport in the soluble form to membrane surfaces. PrPs are expressed in lymphoreticular system and are transported to the brain. Like prions, prionins are found in all TSE diseases. Furthermore, the evolutionary relationship to prions (expressed from the same DNA sequences) and the propensity for binding between different proteins which are expressed from the same DNA sequences (see also Baranyi L. et al., Nature Medicine 1:894-901 (1995)), suggest that prionins may be specific molecule that converts PrPC to PrPSC.

### Expression of prionin proteins.

As stated earlier, certain observations suggest that BSE is caused when cows are inadvertently fed the remains of scrapie infected sheep, and that BSE is transmitted to humans who eat infected meat and this infection produces a disease phenotype which closely resembles CJS. The current invention appears to support this hypothesis, because these three diseases are associated with three species specific unique proteins which potentially can interact with any other PrP, and therefore, can "infect" any cell they might enter.

BSAS SEQ:ID 1, SCRAPAS SEQ ID:NO 2 and CJAS SEQ ID:NO 3. are expressed specifically in cows infected with BSE, sheep infected with scrapie and humans affected with CJS; all three orfs open from identical translation initiation sequences. BSAS and SCRAPAS are positive DNA binding proteins that can activate a number of genes and can infect across species. The latter two proteins are expressed from alternate reading frames within the major prion genes. Hence if cows ingest SCRAPAS in fodder prepared from sheep with scrapie, SCRAPAS might spuriously bind to a PrP in the lymphoid cells located in the stomach, get transported to the brain, activate translation of BSAS which converts more prions to the pathogenic form and hence initiate BSE in these animals. BSAS entering humans by way of the food chain might activate translation (or transcription) of CJAS, which has the same translation initiation sequence as BSAS and SCRAPAS, and initiate the symptoms of CJS.
From the above discussions it should be obvious that prionins can bypass the blood brain barrier chaperoned. Once in the brain they might interact with neuronal membrane binding sites usually occupied by PrP on neuronal membranes. Furthermore since prionins are lipid soluble molecules they can penetrate the plasma membrane, diffuse through lipid layers and enter neurons. Using BSAS we believe that this might work as follow:
The PrP protein in neuronal plasma membrane is a target either specific or opportunistic for prionins. BSAS endogenously expressed in, or entering a cell by an external route, interacts with PrPs. On binding to the PrP the rigid, β-sheet structure of BSAS forces the flexible conformation of the PrP to change in a manner which accommodates the BSAS β- sheet structure. This results in the formation of a prion (see Harrison. S.C., Cell 86:343-344 (1996). The soluble BSAS-Prion complex traverses the cytoplasm and leaves the cell through the membrane, enters the lymph vesicular transport system in which it transverses the blood brain barrier and arrives at the surface of a specific subpopulation of neurons. The latter are the usual targets of PrPs. On contact with a neuron BSAS releases from the prion and penetrates the neuronal membrane. The prion cannot enter the membrane in the converted state and is left in the intraneuronal spaces where aggregation occurs with other discarded prions. Alternatively, on release from BSAS the PrP flips back into the original alpha helical PrP state. The cycle of events is amplified as BSAS that enters the neuron overactivates the expression of PrPs.

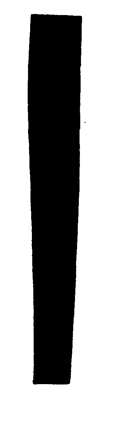

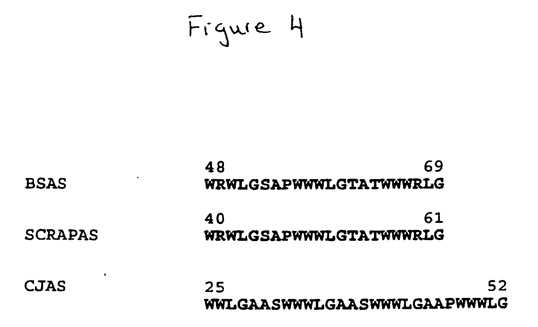

Since BSAS and SCRAPAS contain the identical positive DNA regulatory sequence Figure 4, they are likely to activate similar genes and probably autoactivate their own transcription or translation.

Alternatively, prionins can be expressed from a promoter system located 3' downstream to promoter which is believed to program expression of PrP. Whereas PrPs are expressed in appropriate cells throughout life and appear to be normal essential components of neurons, all be it, with functions presently unknown, (but see Schmerling et al., Cell 93:203-214 (1998)). Prionins are probably expressed, secreted and targeted (specific receptors or opportunistic) to neuronal and other non-neuronal cells only in diseased animals.

For support for the notion that prionins are pathogenic was provided by the finding that prionins are bound to a variety of immunoglobulin molecules in the blood of all animals that are clinically positive for BSE/scrapie and humans with clinical CJD. The antibody was also found in a significant number of clinically negative animals that were exposed to BSE or scrapie. The endogenous antibody was never found in animals that were not exposed to BSE/scrapie nor in clinically normal humans that were not exposed to CJD. This indicates that the subject's immune system considered prionins foreign and tried to neutralize them when they were expressed in animals and humans. The immune response to prionins may be extremely important in the control of TSE diseases especially in controlling cross infections. It is also important in detecting latency and of course in presymptomatic diagnosis of TSE diseases. Furthermore it appears likely that prionin expression can be initiated when the appropriate cells, are exposed to some environmental toxin (e.g., microbial or viral infection, intracellular metabolic toxic by products or stress). Such infections might have the effect of first stressing the subject's immune system in a way that it is unable to cope efficiently with other task and secondly the infecting agent may also activate the expression of prionins. This renders the immune system less effective and permits prionins to escape and enter cells where they interact with PrPs .

In summary, prionins acting as pore forming proteins or acting while bound to the prion in.the neurons disrupts the lipid layer and enter neurons where they activate expression of a number of genes including PrP and prionins. Prionin molecules remaining on or within the membrane elicits a response from the neuronal immune system. This immune response targets cells containing the prionin molecules and hence selectively destroy these prionin bearing cells which takes on the character of an autoimmune reaction against endogenous neurons. Because this autoimmune action selectively targets cells bearing prionins on the surface surrounding cells are for the most part left untouched and the result is the vacoulated spongiform appearance of TSE brains.

### THE USES OF THE MOLECULES OF THE PRESENT INVENTION

### A. Diagnostic Uses

Since BSAS, SEQ ID:NO 1; SCRAPAS SEQ ID:NO 2 and CJAS SEQ ID:NO 3, are not expressed at a detectable level by normal animals or humans, the detection of these molecules in a tissue or fluid sample (such as a biopsy sample, or of blood or urine or saliva) is indicative of the presence of the disease in that subject even before any clinical symptoms are present.

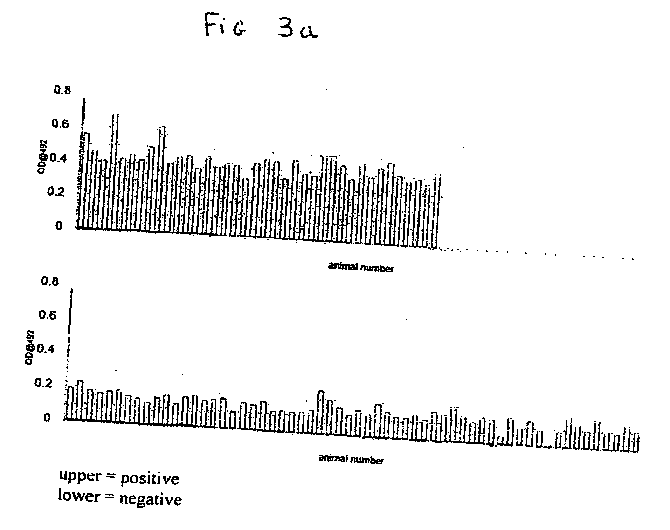

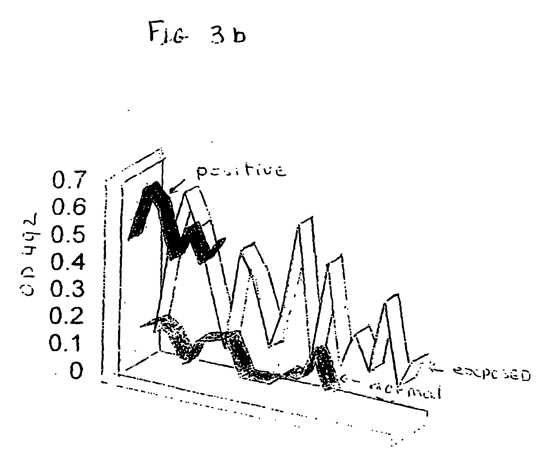

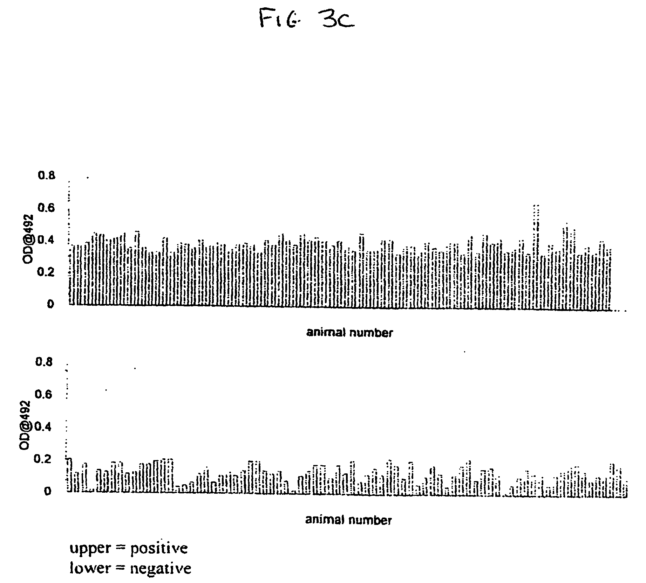

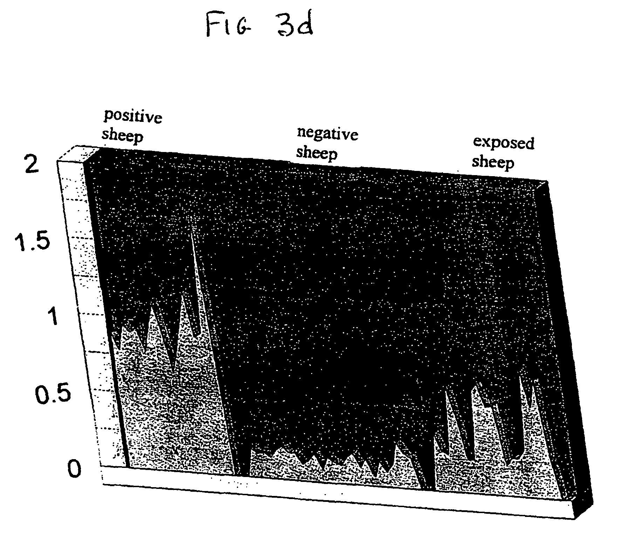

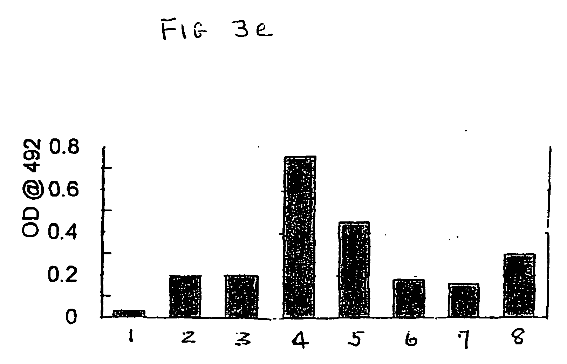

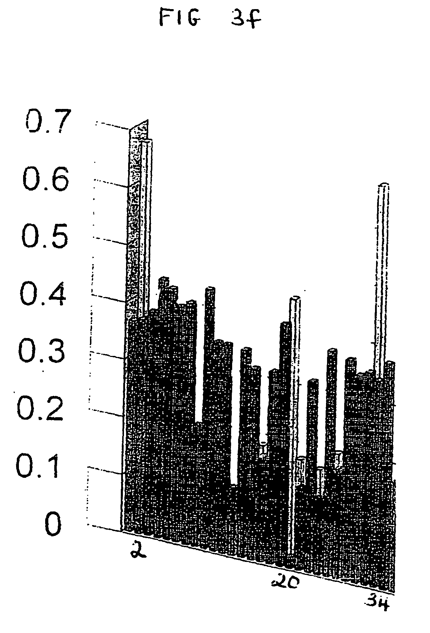

Example 6 : the results of 103, mixed, blinded samples including blood, serum and urine were tested using the ELISA test outlined in Figure 3a. All samples from clinically positive samples tested positive for BSAS or SCRAPAS, all clinically normal animals that were never exposed to BSE or scrapie tested negative for BSAS or SCRAPAS whereas up to 30 % of the clinically negative animals that were exposed to BSE tested positive. The latter animals were presymptomatic for the disease.

We have used a sensitive sandwich ELISA approaches for detecting BSAS, SCRAPAS and CJAS in cow, sheep and human material and for detecting latency and cross contaminations in animals, foods and blood products; however, the detection of these molecules may be done by any of a variety of immunological methods; a large number of suitable immunoassay formats have been described (Yolken, R.H., Rev. Infect. Dis. 4:35 (1982); Collins, W.P., In: Alternative Immunoassays, John Wiley & Sons, NY (1985); Ngo, T.T. et al., In: Enzyme Mediated Immunoassay, Plenum Press, NY (1985).

Example 7. the antibody prepared against the epitope in hamster prionin HAMPAS, SEQ ID:NO 5, can be used to detect CJAS. In lieu of such antibodies, equivalent binding molecules, such as antibody fragments (F(ab'), F(ab')2, single chain antibodies, etc.), recombinant antibodies, chimeric antibodies, etc. may be employed.

As indicated above, immunoassay formats may employ labelled antibodies to facilitate detection. Radioisotopic immunoassays ("RIAs") have the advantages of simplicity, sensitivity, and ease of use. Radioactive labels are of relatively small atomic dimension, and do not normally affect reaction kinetics. Such assays suffer, however, from the disadvantages that, due to radioisotopic decay, the reagents have a short shelf-life, require special handling and disposal, and entail the use of complex and expensive analytical equipment. RIAs are described in Laboratory Techniques and Biochemistry in Molecular Biology, by Work, T.S., et al., North Holland Publishing Company, NY (1978), with particular reference to the chapter entitled "An Introduction to Radioimmune Assay and Related Techniques" by Chard, T.

No single enzyme is ideal for use as a label in every conceivable immunometric assay. Instead, one must determine which enzyme is suitable for a particular assay system. Criteria important for the choice of enzymes are turnover number of the pure enzyme (the number of substrate molecules converted to product per enzyme site per unit of time), purity of the enzyme preparation, sensitivity of detection of its product, ease and speed of detection of the enzyme reaction, absence of interfering factors or of enzyme-like activity in the test fluid, stability of the enzyme and its conjugate, availability and cost of the enzyme and its conjugate, and the like. Examples of suitable enzymes which can be used include peroxidase, acetylcholine esterase, alpha-glycerol phosphate dehydrogenase, alkaline phosphatase, asparaginase, b-galactosidase, catalase, among many others. Peroxidase and urease are among the more preferred enzyme labels, particularly because of chromogenic pH indicators which make its activity readily visible to the naked eye.

### B. Therapeutic Uses

Significantly, the present invention provides means for treating BSE, scrapie and CJS. Such treatment may be either "prophylactic" or "therapeutic." A prophylactic treatment is one that is provided in advance of any clinical symptom of BSE, scrapie or CJS in order to prevent or attenuate any subsequent onset of the disease. A therapeutic treatment is one that is provided in response to the onset of a symptom of BSE, scrapie or CJS and serves to attenuate an actual symptom of the disease.
In one embodiment, such treatment is provided by administering to an animal or human in need of such treatment an effective amount of an antibody, or an antibody fragment (F(ab'), F(ab')2, single chain antibodies, etc.) or a combination of the above that is capable of binding to BSAS, SCRAPAS or CJAS. As used herein, an effective amount is an amount sufficient to mediate a clinically significant change in the severity of a symptom, or a clinically significant delay in the onset of a symptom.

As will be appreciated, for acute administration, monospecific polyclonal or monoclonal antibodies (or fragments of either) may be administered. More preferably, and especially for chronic administration, the use of non-immunogenic antibodies is preferred. Such molecules can be pseudo-homologous (i.e. produced by any species, but altered to a form that is immunologically indistinct from human antibodies). Examples of such pseudo-homologous molecules include "humanized" (i.e. non-immunogenic in a human) prepared by recombinant or other technology. Such antibodies are the equivalents of the monoclonal and polyclonal antibodies, but are less immunogenic, and are better tolerated by the patient.

Humanized anti CJAS can be produced, for example by replacing an immunogenic portion of each antibody with a corresponding, but non-immunogenic portion (i.e. chimeric antibodies) (Robinson, R.R. et al., International Patent Publication PCT/US86/02269; Akira, K. et al., European Patent Application 184, 187; Taniguchi, M., European Patent Application 171,496; Morrison, S.L. et al., European Patent Application 173,494; Neuberger, M.S. et al., PCT Application WO 86/01533; Cabilly, S. et al., European Patent Application 125,023; Better, M. et al., Science 240:1041-1043 (1988) ; Liu, A.Y. et al., Proc. Natl. Acad. Sci. USA 84:3439-3443 (1987); Liu, A.Y. et al., J. Immunol. 139:3521-3526 (1987); Sun, L.K. et al., Proc. Natl. Acad. Sci. USA 84:214-218 (1987); Nishimura, Y. et al., Canc. Res. 47:999-1005 (1987); Wood, C.R. et al., Nature 314:446-449 (1985)); Shaw et al., J. Natl.Cancer Inst. 80:1553 -1559 (1988). General reviews of "humanized" chimeric antibodies are provided by Morrison, S.L. (Science, 229:1202-1207 (1985)) and by Oi, V.T. et al., BioTechniques 4:214 (1986). Suitable "humanized" antibodies can alternatively be produced by CDR or CEA substitution (Jones, P.T. et al., Nature 321:552-525 (1986); Verhoeyan et al., Science 239:1534 (1988); Beidler, C.B. et al., J. Immunol. 141:4053-4060 (1988).

### D. Administration of the Molecules of the Present Invention

Additional pharmaceutical methods may be employed to control the duration of action. Control release preparations may be achieved through the use of polymers to complex or absorb the agents. The controlled delivery may be exercised by selecting appropriate macromolecules (for example polyesters, polyamino acids, polyvinyl pyrrolidone, ethylenevinylacetate, methylcellulose, carboxymethylcellulose, or protamine, sulphate) and the concentration of macromolecules as well as the methods of incorporation in order to control release.

Having now generally described the invention, through references and examples that makes it more readily understood by any one sufficiently skilled in the art, it must be pointed out that these are not intended to be limiting of the present invention, unless specified.

The invention is defined by the claims.

### Table 1

Antigen trap ELISA test results from blood and urine of cows and sheep with BSE and scrapie.
BSE dia = clinical diagnosis of animals
cv = EC country herd
neg = no visible clinical symptoms of BSE but exposed to herds with BSE/scrapie .
pos = clinical symptoms of BSE/scrapie
nz = test material obtained directly Scrapie/BSE free country can 43 -83 = plasma from "40" Canadian cows which were never exposed to BSE * = all samples tested negative in the ELISA.

### Table 2.1 & 2.2

Antibody trap ELISA tests results from serum obtain from scrapie positive sheep and serum obtained from scrapie negative sheep from a flock never exposed to scrapie.

### Table 3

Antibody trap ELISA test results from serum obtained from BSE positive cows and from BSE negative cows that were never exposed to BSE.

### LEGEND TO FIGURES

### Figure #1 a-e

The amino acid sequence of the prionins, BSAS, SCRAPAS, CJAS, MPAS and HAMPAS deduced from the mRNA sequence.

### Figure 2

Sequence of antigenic epitopes used to prepare polyclonal antibodies. epitopes were chemically synthesized using solid state technology, purified by HPLC and coupled to key hole limpet haemocyanin. IgG was purified from immune sera and affinity purified on columns of epitope coupled to CN-sepharose.

### Figure 3

(a) Detection of endogenous anti-BSAS IgG in BSE positive cows but not in well characterised BSE negative cows (plates coated with BSAS and bound antibody detected with anti bovine IgG HRP.
(b) Detection of endogenous anti-BSAS IgG in serum taken from, cows with clinical BSE, clinically normal cows from a herd with cases of BSE and clinically negative cows not exposed to BSE.
(c) Detection of endogenous anti-SCRAPAS IgG in serum taken from scrapie positive sheep but not in serum taken from sheep that were never exposed to scrapie (plates coated with BSAS and anti-SCRAPAS was detected with anti-sheep IgG HRP).
(d) Detection of endogenous anti-SCRAPAS IgG in serum from sheep with clinical scrapie, serum from clinically normal sheep from a flock with several cases of scrapie and serum from sheep never exposed to scrapie.
(e) Detection of CJAS in serum taken from two CJD victims but not in a normal human or in patients with other neurological conditions (plates were coated with anti-SCRAPAS IgG and CJAS was detected with anti-CJAS IgG HRP, (1= physiological aging, 2= diffuse Lewy bodies, 3= Parkinsons, 4= CJD, 5= CJD and Alzheimer's disease, 6= Epilepsy, 7= mixed type dementia.
(f) Detection of endogenous anti-CJAS IgG in serum from two CJD patients but not in serum from 33 clinically normal humans and humans with a variety of neurodegenerative conditions (CJAS epitope was bound to the elisa wells and bound endogenous anti-CJAS IgG was detected with anti human Fc specific IgG HRP) 2 & 34 = CJD, 20= Alzheimer's disease 1, 3-19, 21-33 & 35= normals and other diseases.
g) The presence of BSAS and BSAS complexed to IgG fragments in serum isolated from BSE positive cow. 50 ml of was electrophoresed on cationic non SDS PAGE and blotted unto nylon membrane the blots were treated with anti BSAS IgG and the presence of BSAS IgG complexed to BSAS was detected with a mouse anti rabbit IgG coupled to a chemiluminescent substrate, following exposure to X-ray film. IgG was identified in the band with the complex in a separate experiment not shown.

### Figure 4

a-b sequence of DNA positive regulators of BSAS and SCRAPAS and c. the membrane spanning helix of CJAS.

### Figure 5

Evolutionary relationship between the prionins, BSAS, SCRAPAS and CJAS, MPAS and HAMPAS; between bovine PrP, sheep PrP, human PrP, mouse PrP and hamster PrP.

### Figure 6

The β sheet propensity of the prionins:

**Table 1**

| sample | source | tissue | BSE dia. | scrapie dia. | ELISA |
|---|---|---|---|---|---|
| 1 | cv | serum | neg | na | - |
| 2 | cv | serum | neg | na | + |
| 3 | cv | serum | pos | na | + |
| 4 | cv | serum | neg | na | - |
| 5 | cv | serum | pos | na | + |
| 6 | cv | serum | pos | na | + |
| 7 | cv | serum | pos | na | + |
| 8 | cv | serum | neg | na | + |
| 9 | cv | serum | neg | na | - |
| 10 | cv | serum | pos | na | + |
| 11 | cv | serum | pos | na | + |
| 12 | cv | serum | neg | na | - |
| 13 | cv | serum | pos | na | + |
| 14 | cv | serum | neg | na | - |
| 15 | cv | serum | neg | na | + |
| 16 | cv | serum | neg | na | - |
| 17 | cv | serum | neg | na | + |
| 18 | cv | serum | pos | na | + |
| 19 | cv | serum | neg | na | - |
| 20 | cv | serum | pos | na | + |
| 21 | cv | serum | neg | na | + |
| 22 | cv | serum | neg | na | - |
| 23 | cv | serum | neg | na | - |
| 24 | cv | serum | neg | na | - |
| 25 | cv | serum | neg | na | - |
| 26 | cv | serum | pos | na | + |
| 27 | cv | serum | pos | na | + |
| 28 | cv | serum | pos | na | + + |
| 29 | cv | serum | pos | na | + |
| 30 | cv | serum | pos | na | + |
| 31 | cv | urine | pos | na | + |
| 32 | cv | urine | pos | na | + |
| 33 | cv | urine | pos | na | + |
| 34 | cv | urine | pos | na | + |
| 35 | cv | urine | pos | na | + |
| 36 | cv | urine | neg | na | + |
| 37 | cv | urine | neg | na | + |
| 38 | nz | urine | neg | na | - |
| 39 | nz | urine | neg | na | - |
| 40 | nz | urine | neg | na | - |
| 41 | nz | urine | neg | na | - |
| 42 | nz | urine | neg | na | - |
| 43 - 83 | can | plasma | all neg | na | - |
| 84 | cv | plasma | na | neg | - |
| 85 | cv | plasma | na | neg | - |
| 86 | cv | plasma | na | neg | - |
| 87 | cv | plasma | na | pos | + |
| 88 | cv | plasma | na | neg | - |
| 89 | cv | plasma | na | pos | + |
| 90 | cv | plasma | na | neg | - |
| 91 | cv | plasma | na | pos | + |
| 92 | cv | plasma | na | pos | + |
| 93 | cv | plasma | na | neg | - |
| 94 | cv | plasma | na | neg | - |
| 95 | cv | plasma | na | pos | + |
| 96 | cv | plasma | na | pos | + |
| 97 | cv | plasma | na | pos | + |
| 98 | cv | plasma | na | neg | - |
| 99 | cv | plasma | na | neg | - - |
| 100 | cv | plasma | na | pos | + |
| 101 | cv | plasma | na | : neg | - |
| 102 | cv | plasma | na | pos | + |
| 103 | cv | plasma | na | pos | + + |

**Table 2.1**

| mean absorbance | status | mean absorbance | status |
|---|---|---|---|
| 367 | POS | 211 | NEG |
| 373 | POS | 117 | NEG |
| 389 | POS | 182 | NEG |
| 449 | POS | 19 | NEG |
| 437 | POS | 143 | NEG |
| 409 | POS | 131 | NEG |
| 419 | POS | 189 | NEG |
| 450 | POS | 189 | NEG |
| 362 | POS | 122 | NEG |
| 362 | POS | 127 | NEG |
| 360 | POS | 172 | NEG |
| 331 | POS | 183 | NEG |
| 333 | POS | 204 | NEG |
| 419 | POS | 206 | NEG |
| 331 | POS | 206 | NEG |
| 392 | POS | 40 | NEG |
| 383 | POS | 51 | NEG |
| 360 | POS | 73 | NEG |
| 368 | POS | 117 | NEG |
| 357 | POS | 152 | NEG |
| 391 | POS | 78 | NEG |
| 383 | POS | 111 | NEG |
| 347 | POS | 128 | NEG |
| 378 | POS | 112 | NEG |
| 394 | POS | 138 | NEG |
| 387 | POS | 199 | NEG |
| 333 | POS | 202 | NEG |
| 410 | POS | 147 | NEG |
| 379 | POS | 122 | NEG |
| 453 | POS | 137 | NEG |
| 413 | POS | 80 | NEG |
| 431 | POS | 16 | NEG |
| 414 | POS | 108 | NEG |
| 376 | POS | 139 | NEG |
| 412 | POS | 181 | NEG |
| 366 | POS | 184 | NEG |
| 348 | POS | 10 | NEG |
| 463 | POS | 181 | NEG |
| 350 | POS | 134 | NEG |
| 362 | POS | 213 | NEG |
| 416 | POS | 83 | NEG |
| 417 | POS | 115 | NEG |
| 334 | POS | 164 | NEG |
| 380 | POS | 123 | NEG |
| 380 | POS | 217 | NEG |
| 346 | POS | 177 | NEG |
| 418 | POS | 103 | NEG |
| 373 | POS | 208 | NEG |
| 349 | POS | 65 | NEG |

**Table 2.2**

| | | | |
|---|---|---|---|
| 399 | POS | 115 | NEG |
| 401 | POS | 167 | NEG |
| 331 | POS | 129 | NEG |
| 448 | POS | 48 | NEG |
| 341 | POS | 123 | NEG |
| 473 | POS | 179 | NEG |
| 397 | FOS | 223 | NEG |
| 426 | POS | 90 | NEG |
| 352 | POS | 165 | NEG |
| 368 | POS | 167 | NEG |
| 433 | POS | 116 | NEG |
| 342 | POS | 5 | NEG |
| 598 | POS | 58 | NEG |
| 334 | POS | 104 | NEG |
| 397 | POS | 158 | NEG |
| 358 | POS | 129 | NEG |
| 538 | POS | 120 | NEG |
| 512 | POS | 58 | NEG |
| 336 | POS | 118 | NEG |
| 389 | POS | 153 | NEG |
| 349 | POS | 179 | NEG |
| 433 | POS | 188 | NEG |
| 375 | POS | 146 | NEG |
| | | 92 | NEG |
| | | 119 | NEG |
| | | 123 | NEG |
| | | 208 | NEG |
| | | 184 | NEG |
| | | 105 | NEG |

**Table 3**

| Mean absorbance | Status | | |
|---|---|---|---|
| 669 | POS | 118 | NEG |
| 455 | POS | 33 | NEG |
| 684 | POS | 41 | NEG |
| 518 | POS | 268 | NEG |
| 572 | POS | 153 | NEG |
| 546 | POS | 137 | NEG |
| 453 | POS | 101 | NEG |
| 474 | POS | 121 | NEG |
| 668 | POS | 83 | NEG |
| 457 | POS | 74 | NEG |
| 588 | POS | 66 | NEG |
| 572 | POS | 78 | NEG |
| 813 | POS | 200 | NEG |
| 579 | POS | 191 | NEG |
| 496 | POS | 89 | NEG |
| 515 | POS | 120 | NEG |
| 589 | POS | 111 | NEG |
| 60 | NEG | 82 | NEG |
| 14 | NEG | 187 | NEG |
| 44 | NEG | 238 | NEG |
| 22 | NEG | 114 | NEG |
| 206 | NEG | 41 | NEG |
| 22 | NEG | 110 | NEG |
| 53 | NEG | 70 | NEG |
| 214 | NEG | 239 | NEG |
| 31 | NEG | 41 | NEG |
| 22 | NEG | 118 | NEG |
| 196 | NEG | | |
| 98 | NEG | | |
| 188 | NEG | | |
| 259 | NEG | | |
| 39 | NEG | | |
| 179 | NEG | | |
| 96 | NEG | | |
| 169 | NEG | | |
| 133 | NEG | | |
| 204 | NEG | | |
| 89 | NEG | | |
| 152 | NEG | | |
| 41 | NEG | | |
| 102 | NEG | | |
| 30 | NEG | | |
| 111 | NEG | | |
| 103 | NEG | | |
| 105 | NEG | | |
| 290 | NEG | | |
| 172 | NEG | | |
| 131 | NEG | | |
| 147 | NEG | | |
| 58 | NEG | | |

## Claims

1. A protein molecule free of natural contaminants selected from the group consisting of BSAS, SCRAPAS, CJAS, MPAS and HAMPAS the sequence of which is SEQ ID NO:1, SEQ ID NO:2, SEQ ID NO:3, SEQ ID NO:4 and SEQ ID NO:5, respectively.

2. An antibody specific for either BSAS, SCRAPAS, CJAS or HAMPAS, the sequence of which is SEQ ID NO:1, SEQ ID NO:2, SEQ ID NO:3 and SEQ ID NO:5, respectively.

3. An antibody of claim 2, wherein the antibody is produced by immunizing appropriate animals with polypeptide domains of BSAS or SCRAPAS or CJAS or HAMPAS, respectively.

4. An antibody of claim 2 specific for either BSAS, SCRAPAS or CJAS, wherein said antibody is produced endogenously by a cow or by a sheep or by a human, respectively.

5. An antibody, or an antibody fragment, or single chain antibodies, or a combination thereof, capable of binding specifically to either BSAS, SCRAPAS or CJAS, for use in the prophylactic or therapeutic treatment of a transmissible spongiform encephalopathy (TSE) in humans or animals.

6. Use of an antibody, or of an antibody fragment, or of single chain antibodies, or of a combination thereof, capable of binding specifically to either BSAS, SCRAPAS or CJAS for the manufacture of a medicament for the prophylactic or therapeutic treatment of a TSE in humans or animals.

7. An antibody as defined in claim 5 or a use as defined in claim 6, wherein the antibody is a monospecific polyclonal antibody or a monoclonal antibody or a recombinant antibody or a humanized antibody.

8. A method for the diagnosis or the presymptomatic diagnosis of a TSE in humans or animals comprising detecting in a tissue or fluid sample a protein as defined by SEQ ID NO:1, SEQ ID NO:2, or SEQ ID NO:3.

9. A method for the diagnosis or the presymptomatic diagnosis of a TSE in humans or animals comprising detecting in a sample an antibody specific for any of the proteins as defined by SEQ ID NO:1, SEQ ID NO:2, or SEQ ID NO:3.

10. An antibody as defined in claims 5 or 7 or a use as defined in claims 6 or 7 or a method as defined in claims 8 or 9, wherein the TSE is bovine spongiform encephalopathy (BSE), scrapie disease or Creutzfeldt-Jakob Syndrome (CJS), respectively.

## Patentansprüche

1. Ein Protein Molekül, frei von natürlichen Kontaminationen, ausgewählt aus einer Gruppe bestehend aus BSAS, SCRAPAS, CJAS, MPAS und HAMPAS, deren Sequenzen die SEQ ID NR: 1, SEQ ID NR: 2, SEQ ID NR: 3, SEQ ID NR: 4 und SEQ ID NR: 5 sind.

2. Ein Antikörper spezifisch für entweder BSAS, SCRAPAS, CJAS oder HAMPAS, deren Sequenzen die SEQ ID NR: 1, SEQ ID NR: 2, SEQ ID NR: 3, SEQ ID NR: 4 und SEQ ID NR: 5 sind.

3. Ein Antikörper von Anspruch 2, worin der Antikörper durch Immunisierung eines geeigneten Tieres mit Polypeptid-Domänen von BSAS oder SCRAPAS oder CJAS oder HAMPAS hergestellt ist.

4. Ein Antikörper von Anspruch 2 mit Spezifität für entweder BSAS, SCRAPAS oder CJAS, worin besagter Antikörper endogen durch entweder eine Kuh, ein Schaf oder einen Menschen produziert wird.

5. Ein Antikörper oder ein Antikörper-Fragment oder einkettige Antikörper, oder Kombinationen von diesen, fähig, spezifisch entweder an BSAS, SCRAPAS oder an CJAS zu binden, für der Gebrauch zur prophylaktischen oder therapeutischen Behandlung von übertragbaren spongiformen Enzephalopathien (TSE) bei Menschen oder bei Tieren.

6. Der Gebrauch eines Antikörpers oder eines Antiköprer-Fragmentes oder eines einkettigen Antikörpers, oder von Kombinationen von ihnen, fähig, spezifisch entweder an BSAS, SCRAPAS oder an CJAS zu binden, für die Herstellung eines Medikaments zur prophylaktischen oder therapeutischen Behandlung von TSE bei Menschen oder Tieren.

7. Ein Antikörper wie definiert in Anspruch 5 für den Gebrauch wie definiert in Anspruch 6, worin der Antikörper ein monospezifischer polyklonaler Antikörper oder ein monoklonaler Antikörper oder ein rekombinanter Antikörper oder ein humanisierter Antikörper ist.

8. Eine Methode zur Diagnose oder präsymptomatischen Diagnose von TSE bei Menschen oder Tieren, die den Nachweis eines Proteins wie definiert durch SEQ ID NR: 1, SEQ ID NR: 2 oder SEQ ID NR: 3 in einer Gewebe- oder Körperflüssigkeitsprobe umfasst.

9. Eine Methode zur Diagnose oder präsymptomatischen Diagnose von TSE bei Menschen oder Tieren, die den Nachweis eines Antikörpers mit Spezifität für jeweils eines der in SEQ ID NR: 1, SEQ ID NR: 2 oder SEQ ID NR: 3 definierten Proteine in einer Probe umfasst.

10. Ein Antikörper wie definiert in Ansprüchen 5 oder 7, oder der Gebrauch wie definiert in Ansprüchen 6 oder 7, oder eine Methode wie definiert in Anprüchen 8 oder 9, worin die TSE bovine spongiforme Enzephalophathie (BSE), Skrapie oder Creutzfeldt-Jakob Syndrom (CJS) ist.

## Revendications

1. Une protéine sans contaminant endogène sélectionnée dans le groupe comprenant la BSAS, la SCRAPAS, la CJAS, la MPAS et la HAMPAS dont les séquences sont, dans le même ordre, SEQ ID NO : 1, SEQ ID NO : 2, SEQ ID NO : 3, SEQ ID NO : 4 et SEQ ID NO : 5.

2. Un anticorps spécifique de la BSAS, de la SCRAPAS, de la CJAS ou de la HAMPAS dont les séquences sont SEQ ID NO : 1, SEQ ID NO : 2, SEQ ID NO : 3 et SEQ ID NO : 5.

3. Un anticorps tel que revendiqué en 2., produit par immunisation de l'animal approprié au moyen d'un domaine polypeptidique de la BSAS, de la SCRAPAS, de la CJAS ou de la HAMPAS.

4. Un anticorps spécifique de la BSAS, de la SCRAPAS ou de la CJAS tel que revendiqué en 2., produit de façon endogène, dans le même ordre, chez le bovin, l'ovin ou l'humain.

5. Un anticorps, un fragment d'anticorps, des anticorps mono-chaînes ou une combinaison de ceux-ci, pouvant se lier de façon spécifique à la BSAS, à la SCRAPAS ou à la CJAS, destiné au traitement thérapeutique ou prophylactique d'une encéphalopathie spongiforme transmissible (EST) chez l'humain ou chez l'animal.

6. L'utilisation d'un anticorps, d'un fragment d'anticorps, d'anticorps mono-chaînes ou d'une combinaison de ceux-ci, pouvant se lier de façon spécifique à la BSAS, à la SCRAPAS ou à la CJAS, destiné à la fabrication d'un médicament pour le traitement thérapeutique ou prophylactique d'une EST chez l'humain ou chez l'animal.

7. Un anticorps tel que défini dans la revendication 5. ou une utilisation telle que définie dans la revendication 6. lorsque l'anticorps est un anticorps polyclonal monospécifique, un anticorps monoclonal, un anticorps recombinant ou un anticorps humanisé.

8. Une méthode de diagnostic ou de diagnostic présymptomatique d'une EST humaine ou animale qui inclut la détection d'une protéine **caractérisée par** la SEQ ID NO : 1, la SEQ ID NO : 2 ou la SEQ ID NO : 3 dans un échantillon de tissu ou de fluide.

9. Une méthode de diagnostic ou de diagnostic présymptomatique d'une EST humaine ou animale qui inclut la détection d'un anticorps spécifique de n'importe quelle protéine **caractérisée par** la SEQ ID NO : 1, la SEQ ID NO : 2 ou la SEQ ID NO : 3 dans un échantillon.

10. Un anticorps tel que défini dans la revendication 5 ou 7 ou une utilisation telle que définie dans la revendication 8 ou 9 lorsque l'EST est l'encéphalopathie spongiforme bovine (ESB), la tremblante ou la maladie de Creutzfeldt-Jacob.


### SEQUENCE LISTING

(1) GENERAL INFORMATION:
   (i) APPLICANT: Bergmann, Johanna & Preddie, Rick.E
   (ii) TITLE OF INVENTION: "PRIONINS" HIGHLY SPECIFIC TARGETS FOR NONINVASIVE PRESYMPTOMATIC DETECTION, MAP BASED VACCINES AND CONTROL OF CROSSINFECTION OF FOODS AND BLOOD PRODUCTS IN TSE DISEASES.
   (iii) NUMBER OF SEQUENCES: 5
   (iv) CORRESPONDENCE ADDRESS:
      (A) ADDRESSEE: DR. J. BERGMANN
      (B) STREET: MÖRIKESTR. 22
      (C) CITY: HAMBURG
      (D) STATE:
      (E) COUNTRY: GERMANY
      (F) ZIP: 22587
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: NONE
      (B) REGISTRATION NUMBER:
   (ix) TELECOMMUNICATION INFORMATION:
      (A) TELEPHONE: (4940) 862-576
      (B) TELEFAX: (4940) 862-596
(2) INFORMATION FOR SEQ ID NO:1:
   (i) SEQUENCE CHARACTERISTICS:
      (A) LENGTH: 81 amino acids
      (B) TYPE: protein
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (A) ORGANISM: Bovine
   (vii) IMMEDIATE SOURCE:
      (B) CLONE: BSAS
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 64 amino acids
      (B) TYPE: amino acid
      (D) TOPOLOGY: linear
   (ii) MOLECULE TYPE: protein
   (iii) HYPOTHETICAL: NO
   (vi) ORIGINAL SOURCE:
      (A) ORGANISM: Sheep
   (vii) IMMEDIATE SOURCE:
      (B) CLONE: Scrapas
   (xi) SEQUENCE DESCRIPTION: 2:
(2) INFORMATION FOR SEQ ID NO:3:
   (i) SEQUENCE CHARACTERISTICS:
      (A) LENGTH: 65 amino acids
      (B) TYPE: amino acid
      (D) TOPOLOGY: linear
   (ii) MOLECULE TYPE: protein
   (iii) HYPOTHETICAL: NO
   (vi) ORIGINAL SOURCE:
      (A) ORGANISM: homo sapiens
   (vii) IMMEDIATE SOURCE:
      (B) CLONE: CJAS
   (xi) SEQUENCE DESCRIPTION: SEQ ID NO:3:
   Met Glu His Trp Gly Gln Pro Ile Pro Gly Ala Gly Gln Pro Trp
                    5                  10                  15
   Arg Gln Pro Leu Pro Thr Ser Gly Arg Trp Trp Leu Gly Ala Ala
                   20                  25                  30
   Ser Trp Trp Trp Leu Gly Ala Ala Ser Trp Trp Trp Leu Gly Ala
                   35                  40                  45
   Ala Pro Trp Trp Trp Leu Gly Ser Arg Arg Trp His Pro Gln Ser
                   50                  55                  60
   Val Glu Gln Ala Glu
                   65
(2) INFORMATION FOR SEQ ID NO:4:
   (i) SEQUENCE CHARACTERISTICS:
      (A) LENGTH: 52 amino acids
      (B) TYPE: protein
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (A) ORGANISM: Mouse
   (vii) IMMEDIATE SOURCE:
      (B) CLONE: MPAS
   (xi) SEQUENCE DESCRIPTION: SEQ ID NO:4:
   Met Gly Ala Ala Gly Asp Asn Leu Met Val Val Val Gly Val Ser
                   5                   10                  15
   Pro Met Ala Val Asp Gly Ala Lys Glu Gly Val Pro Ile Ile Ser
                   20                  25                  30
   Gly Thr Ser Pro Ala Asn Gln Lys Pro Thr Ser Ser Ile Trp Gln
                   35                  40                  45
   Gly Leu Arg Gln Leu Gly Gln
                   50
(2) INFORMATION FOR SEQ ID NO:5:
   (i) SEQUENCE CHARACTERISTICS:
      (A) LENGTH: 39 amino acids
      (B) TYPE: protein
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (A) ORGANISM: hamster
   (vii) IMMEDIATE SOURCE:
      (B) CLONE: HAMPAS
   (xi) SEQUENCE DESCRIPTION: SEQ ID NO:5:
   Met Gly Thr Ala Pro Trp Trp Trp Leu Gly Thr Thr Ser Trp Trp
                    5                   10                  15
   Trp Leu Gly Ser Ala Pro Trp Trp Trp Leu Gly Ser Arg Arg Trp
                   20                   25                  30
   His Pro Gln Ser Val Glu Gln Ala Gln
                   35